(19) Europäisches Patentamt
European Patent Office
Office européen des brevets

(11) Publication number: **0 030 140**
**A2**

(12) # EUROPEAN PATENT APPLICATION

(21) Application number: 80304285.2

(22) Date of filing: 28.11.80

(51) Int. Cl.[3]: C 07 D 251/44
C 07 D 251/46, C 07 D 251/16
C 07 D 251/42, C 07 D 239/42
C 07 D 239/46, C 07 D 239/52
C 07 D 251/18, C 07 D 251/52
C 07 D 239/48, C 07 D 239/56

(30) Priority: 30.11.79 US 98722
30.11.79 US 98725
10.10.80 US 192681
14.10.80 US 193190

(43) Date of publication of application:
10.06.81 Bulletin 81/23

(84) Designated Contracting States:
BE DE FR GB IT LU NL

(71) Applicant: E.I. DU PONT DE NEMOURS AND COMPANY
Legal Department 1007 Market Street
Wilmington, Delaware 19898(US)

(72) Inventor: Reap, James John
2505 Bona Road
Wilmington Delaware 19810(US)

(72) Inventor: Roche, Robert Thomas
5 Marie Court
Elmwood Newark Delaware 19713(US)

(72) Inventor: Yeh, Chin-Lung
116 Weldin Park Drive
Wilmington Delaware 19803(US)

(74) Representative: Wardrope, Alexander James
Brownlie et al,
Frank B. Dehn & Co. Imperial House 15-19 Kingsway
London WC2B 6UZ(GB)

(54) **Processes for preparing herbicidal N-(substituted heterocyclicaminocarbonyl)-aromatic sulfonamides.**

(57) The invention provides a process for the preparation of a N-(haloheterocyclic-aminocarbonyl) aromatic sulfonamide of formula

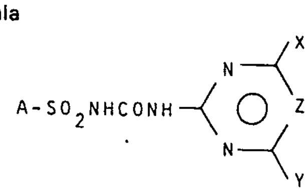

(IV)

(wherein

A is an optionally substituted homocyclic or heterocyclic aromatic group;

X is H; Cl; Br; $C_1$-$C_2$ alkyl; $CF_3$; $CH_3O$; methoxymethyl or $-CH_2CH_2OCH_3$;

Y is Cl or Br; and

Z is CH, N, C-Cl or C-$CH_3$)

characterised in that an aromatic sulfonamide A-$SO_2NH_2$ (wherein A is as defined above) is contacted with an isocyanate of formula

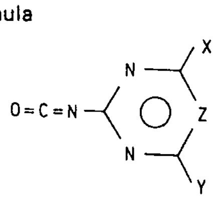

(V)

(wherein X, Y and Z are as defined above). The resulting compounds may be further contacted with selected nucleophiles to yield further N-(substituted heterocyclic-aminocarbonyl) aromatic sulfonamides.

EP 0 030 140 A2

0030140

# Processes for Preparing Herbicidal N-(substituted heterocyclic-aminocarbonyl)-aromatic sulfonamides

## Background of the Invention

This invention relates to a new process for preparing N-(haloheterocyclic-aminocarbonyl)aromatic sulfonamides and N-(substituted heterocyclic-aminocarbonyl)aromatic sulfonamides. These compounds are useful as herbicides and also as intermediates for preparing herbicides.

U.S. Patent 4,169,719 reports certain N-(alkoxyheterocyclic-aminocarbonyl)aromatic sulfonamides as herbicides. This patent teaches a method of preparing these herbicides involving the reaction of an arylsulfonyl isocyanate with an alkoxyheterocyclic amine:

$$ArSO_2NCO + H_2N\text{-(alkoxyheterocycle)} \longrightarrow ArSO_2NHCONH\text{(alkoxyheterocycle)}$$

Due to the increasing need for inexpensive and effective herbicides, there is a definite need for new and efficient processes for preparing these herbicides.

According to one aspect of the invention there is provided a process for preparing a herbicidal compound of formula

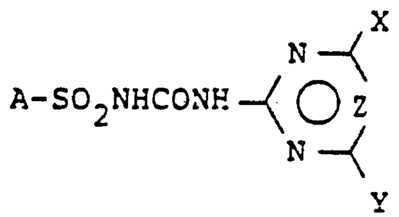

(IV)

(wherein

A is an optionally substituted homocyclic or heterocyclic aromatic group;

X is H; Cl; Br; $C_1$-$C_2$ alkyl; $CF_3$; $CH_3O$; methoxymethyl or $-CH_2CH_2OCH_3$;

Y is Cl or Br; and

Z is CH, N, C-Cl or C-$CH_3$)

characterised in that an aromatic sulfonamide A-$SO_2NH_2$ (wherein A is as defined above) is contacted with an isocyanate of formula

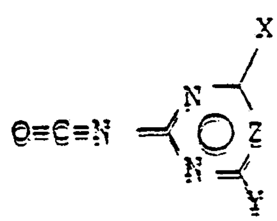

(V)

(wherein X, Y and Z are as defined above).

This process may be illustrated by the following equation:

1) 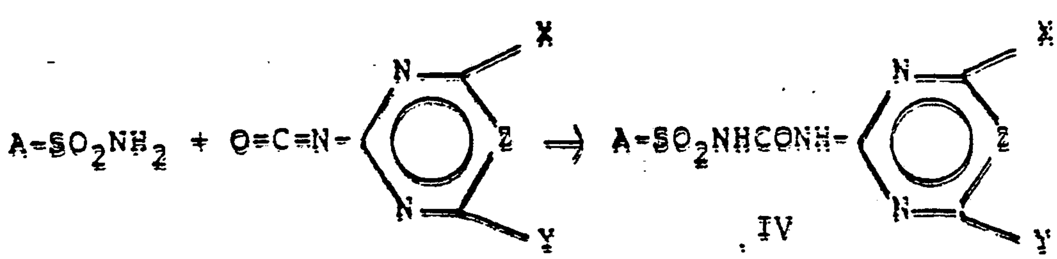

IV

where

A, X, Y and Z are as previously defined.

The product N-(haloheterocyclicamino-carbonyl) aromatic sulfonamides (IV) may be further reacted with selected nucleophiles to yield other herbicidal N-(substituted heterocyclicaminocarbonyl)-aromatic sulfonamides of the formulae:

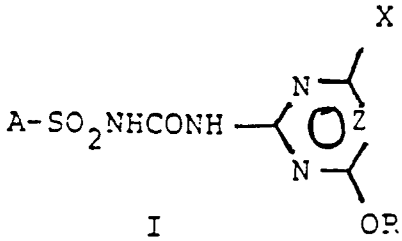

I

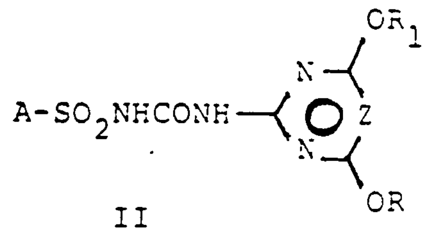

II

and

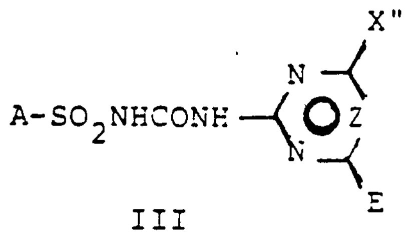

III

wherein

A is an optionally substituted homocyclic or heterocyclic aromatic group;

X is H, Cl, Br, $C_1$-$C_2$ alkyl, $CF_3$, $CH_3O$, methoxymethyl or $-CH_2CH_2OCH_3$;

X" is $C_1$-$C_2$ alkyl, $CF_3$, methoxymethyl, $CH_3O$ or $-CH_2CH_2OCH_3$;

E is $-NR_2R_3$ or $C_1$-$C_6$ alkylthio;

R is methyl, ethyl, $-CH(CH_3)-CO_2CH_3$ or $-CH_2CO_2CH_3$;

$R_1$ is methyl or ethyl;

$R_2$ and $R_3$ are independently $C_1$-$C_4$ alkyl; and

Z is CH, N, C-Cl or C-$CH_3$.

The processes for preparing compounds of Formulas I, II and III may be illustrated by the following three equations:

2) 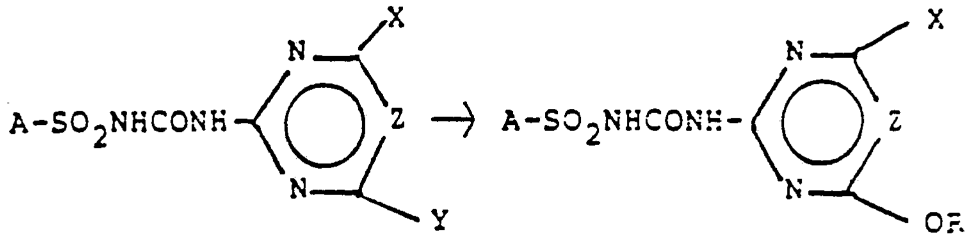

IV I

3) 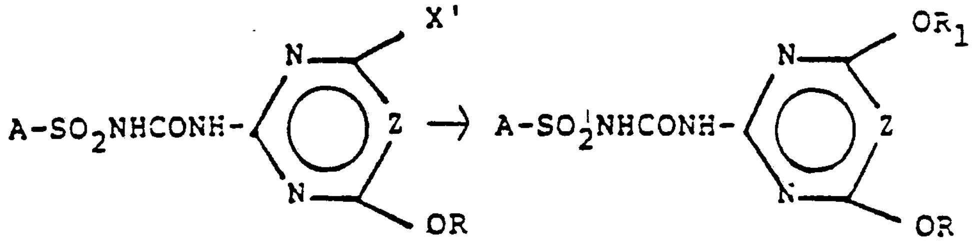

I-a II

4) 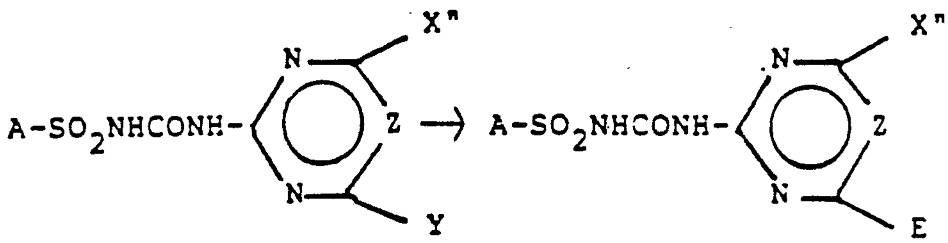

IV-a III

where

A, X, X", E, R, $R_1$, and Z are as previously defined;

X' is Cl or Br; and

Y is Cl or Br.

## Detailed Description of the Invention

In Formulas I, II, III and IV above, A is an optionally substituted homocyclic or heterocyclic aromatic group. Homocyclic aromatic groups include substituted phenyl and naphthyl. Heterocyclic aromatic groups include furanyl, thienyl and pyridyl. A may be any of, but is not limited to, the following groups:

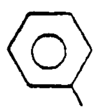 , 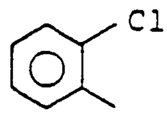 , 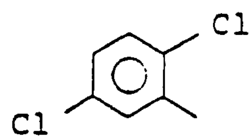 , 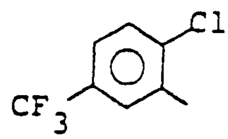 ,

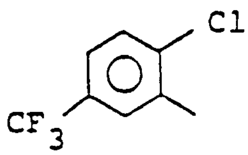 , 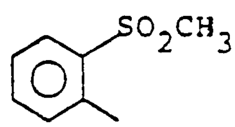 , 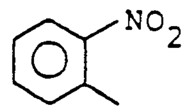 , 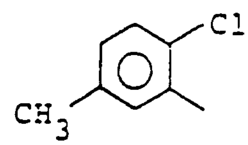 ,

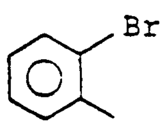 , 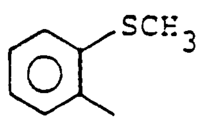 , 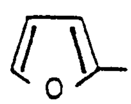 , 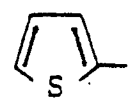 ,

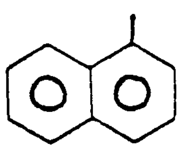 , 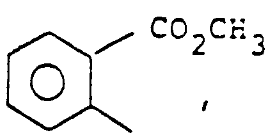 , 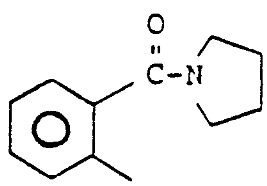 ,

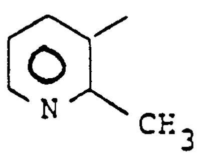 , 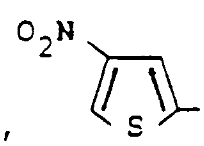 ,

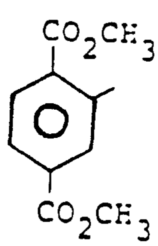 , 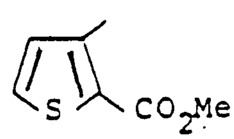

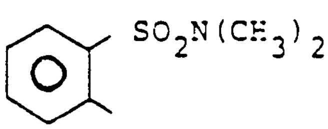 and 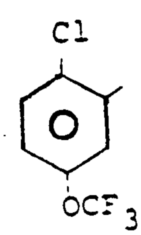

Preferred definitions of A, due to the high herbicidal activity of the product compounds, are the following:

ortho-substituted phenyl
naphthyl
2-furanyl
2-thienyl
3-thienyl
2-substituted-3-thienyl
4-substituted-3-thienyl
pyridyl
2-substituted-3-pyridyl

The aromatic substituents represented by A may optionally be substituted at any position with any substituent unreactive with isocyanate. Examples of possible substituents are those shown in the figures above. Compounds of Formulas I,II, III and IV, in which A is phenyl substituted at the ortho-position with carboxylic acid or carboxylic acid amide, more preferably with the latter and most preferably with pyrrolidine carboxamide show impressive herbicidal activity.

Reaction Step (1)

In the reaction illustrated in Equation 1), an aromatic sulfonamide is contacted with a heterocyclic isocyanate to yield an N-(haloheterocyclicaminocarbonyl)aromatic sulfonamide of Formula IV.

The aromatic sulfonamides used in this reaction may be prepared by one or more methods known in the art. The preparation of aromatic sulfonamides from ammonium hydroxide and arylsulfonyl chloride is widely reported in the literature, e.g., Crossley et al., J. Am. Chem. Soc. 60, 2223 (1938). The preparation of furansulfonamides is described in J. Org. Chem. 18, 894 (1953). Pyridine sulfonamides are described by H. L. Tale in "Pyridine and Its Derivatives" Supplement, Part 4 (1975). Thiophenesulfonamides are prepared according to the references cited in "Thiophene and Its Derivatives", H. D. Hartough, Interscience, New York (1952) and A. H. Blatt et al., J. Am. Chem. Soc. 79, 1693 (1957). All of these articles are herein incorporated by reference. The preparation of other homocyclic and heterocyclic aromatic suflonamides is also reported in the literature and would be known to one skilled in the art.

The heterocyclic isocyanates used in this process may be prepared according to methods described in Swiss Patent 579,062, U.S. Patent 3,919,228, U.S. Patent 3,732,223, and Angew. Chem. Int. Ed. 10, 402 (1976), the disclosures of which are herein incorporated by reference.

The aromatic sulfonamide and the heterocyclic isocyanate are contacted in the presence of an inert organic solvent, for example, acetonitrile, tetrahydrofuran (THF), toluene, acetone or butanone. Optionally, a catalytic amount of a base, such as 1,4-diazabicyclo[2.2.2]octane (DABCO),

potassium carbonate, sodium hydride or potassium *tert*-butoxide, may be added to the reaction mixture. The quantity of base constituting a catalytic amount would be obvious to one skilled in the art. The reaction mixture is preferably maintained at a temperature of about 25 to 110°C, and the product can generally be recovered by cooling and filtering the reaction mixture. For reasons of efficiency and economy, the preferred solvents are acetonitrile and THF, and the preferred temperature range is about 60 to 85°C.

For reasons of ease of synthesis and/or lower cost, the preferred products of the claimed process are those compounds of Formula IV where, independently, X is Cl or Br, Y is Cl and Z is CH or N. More preferred for the same reasons are those compounds where X and Y are simultaneously Cl.

Reaction Steps (2) and (3)

In Reaction Steps (2) and (3), one or two of the halogen atoms on the heterocyclic ring of the compound of Formula IV is displaced by a nucleophilic species. Generally, this may be done by contacting the compound of Formula IV with alkoxide, -OR, where R is as defined above.

It is preferred to contact the compound of Formula IV with at least two equivalents of alkoxide, -OR. The alkoxide can be provided in number of ways:

(a) The compound of Formula IV can be suspended or dissolved in an alkanol solvent, ROH, in the presence of at least two equivalents of alkoxide, -OR. The alkoxide can be added directly as alkali metal or alkaline earth metal alkoxide or

can be generated by the addition to the alkanol solvent of at least two equivalents of a base capable of generating alkoxide from the solvent. Suitable bases include, but are not limited to, the alkali and alkaline earth metals, their hydrides and tert-butoxides. For example, when R is methyl, the compound of Formula IV could be suspended or dissolved in methanol in the presence of two equivalents of sodium methoxide. Alternatively, two equivalents of sodium hydride could be used in place of the sodium methoxide.

(b) The compound of Formula IV can be suspended or dissolved in an inert solvent in the presence of at least two equivalents of alkoxide, -OR. Suitable inert solvents include, but are not limited to, acetonitrile, THF and dimethylformamide. The alkoxide may be added directly as alkali metal or alkaline earth metal alkoxide or may be generated from alkanol and a base as described in (a) above. For example, when R is methyl, the compound of Formula IV could be suspended or dissolved in THF in the presence of two equivalents of sodium methoxide. Alternatively, two equivalents each of methanol and sodium hydride could be used instead of sodium methoxide.

For reasons of economy and efficiency, procedure (a) is the more preferred method.

It should be noted that two equivalents of alkoxide are required for Reaction Step (2). This is due to the reaction which is believed to occur between the alkoxide and the sulfonyl nitrogen of the sulfonamide of Formula IV. When alkoxide is used, the first equivalent of alkoxide removes a proton from the sulfonyl nitrogen, and it is only the second equivalent which effects displacement of the halogen. As a result, two equivalents of alkoxide are required. The resulting salt must be acidified, e.g., with sulfuric, hydrochloric or acetic acid, to yield a compound of Formula I. Applicant, of course, does not intend to be bound by the mechanism described above.

In Reaction Step (3) a compound of Formula I-a, substituted with at least one displaceable group, is contacted with two equivalents of alkoxide, $-OR_1$, where $R_1$ is as described above. The compound of Formula I-a is prepared according to Reaction Step (2) from a compound of Formula IV where X is Cl or Br. The alkoxide, $-OR_1$, may be provided in either of the methods described above in connection with Reaction Step (3), and the resulting salt can be acidified to yield a compound of Formula II.

When $R=R_1$, Reaction Steps (2) and (3) may be combined. Thus, a compound of Formula IV may be contacted with at least three equivalents of alkoxide, -OR.

When a compound of Formula IV contains two displaceable groups, e.g., both X and Y are Cl or Br, certain reaction conditions will favor displacement of only one of the group. These conditions are the use of low temperatures and the slow addition of the stoichiometric amount of alkoxide or alkoxide-generating base to the medium containing the compound of Formula IV.

Both reaction Steps (2) and (3) are preferably run at temperatures within the range of about -10° to 80°C, the range of about 0° to 50°C being more preferred.

Due to ease of synthesis and/or lower cost, the preferred products of Reaction Steps (2) and (3) are those compounds of Formula I and Formula II where Z is CH or N. More preferred are those preferred compounds of Formulas I and II where X is $C_1-C_2$ alkyl and R is methyl or ethyl. Most preferred are those more preferred compounds where $R=R_1$.

Reaction Step (4)

Reaction Step (4) involves the displacement of the halogen atom in a compound of Formula IV-a by dialkylamino or alkylthio nucleophiles. The starting material, a compound of Formula IV-a, is prepared as in Equation (1), and X" is limited to $C_1-C_2$ alkyl, $CF_3$, $CH_3O$, 2-methoxyethyl and methoxymethyl.

For this reaction, the compound of Formula IV-a is suspended or dissolved in an inert solvent, such as acetonitrile or THF. At least one equivalent of the nucleophilic species and at least two equivalents of a base are then contacted with the starting material. The first equivalent of base is believed to neutralize the sulfonamido proton. The second equivalent of base generates mercaptide or $-NR_2R_3$ from the mercaptan or the dialkylamine. Suitable bases include sodium hydride, sodium methoxide and sodium hydroxide.

Suitable reaction temperatures are within the range of about -10° to 80°C, with a range of about 0° to 50° being preferred. The product may be isolated by dilution of the reaction mixture with water, mild acidification and filtration.

Due to ease of synthesis and/or lower cost, the preferred products of Reaction Step (4) are those compounds of Formula III where X" is methyl or ethyl, E is $CH_3S$, $CH_3CH_2S$, $(CH_3)_2N$ or $(CH_3CH_2)_2N$, and Z is CH or N.

The following examples are provided to more fully illustrate the invention but are not to be construed as limiting the scope thereof. Unless otherwise indicated, percentages are by weight and temperatures are in degrees centigrade.

Example 1

N-[(4,6-dichloro-1,3,5-triazin-2-yl)-aminocarbonyl]benzenesulfonamide

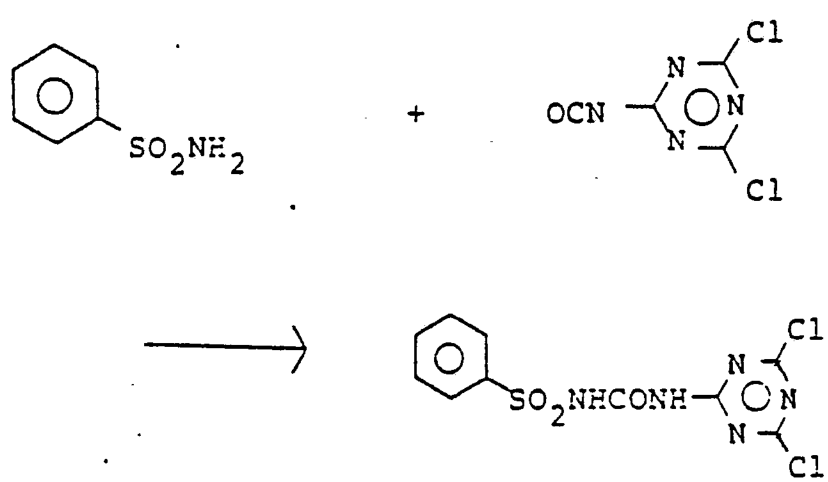

1a

To 3.0g (0.02 mole) of benzenesulfonamide in 20 ml of acetonitrile was added 4.0g (0.022 mole) of dichloro-s-triazinyl isocyanate. The mixture was boiled under reflux for 1/2 hour, during which a white suspension formed. The mixture was cooled to room temperature and filtered. The resulting solid was washed with diethyl ether to yield 6.8g of compound 1a, a white solid with a melting point of 200-202°.

## Example 2

1-/2-{[(4,6-dichloro-1,3,5-triazin-2-yl)-aminocarbonyl]aminosulfonyl } benzoyl/pyrrolidine

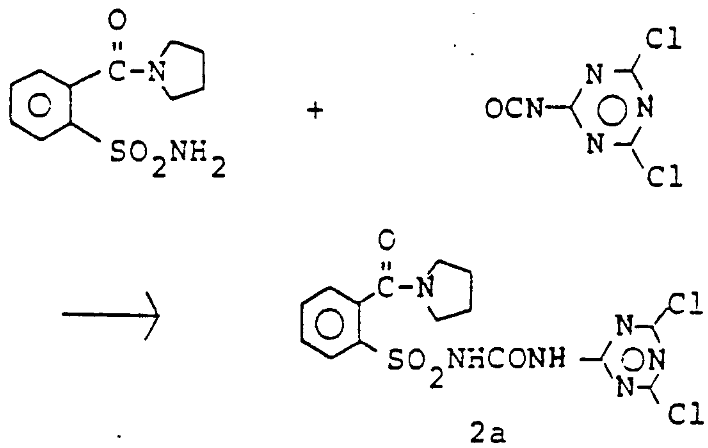

2a

To 2.5g (0.01 mole) of the sulfonamide in 15 ml acetonitrile was added 2.1g (0.011 mole) of dichloro-s-triazinyl isocyanate. The reaction mixture was boiled under reflux for one hour and then cooled to room temperature. Filtration of the solution yielded 4.0g of compound 2a, a white solid with a melting point of 181-183°.

According to the process of this invention, the following compounds can be made. This list is exemplary only.

Table I

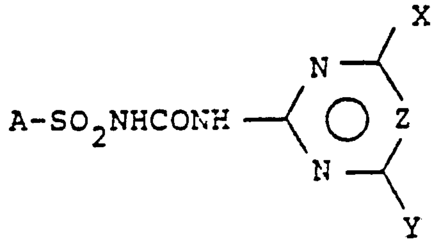

| A | Z | X | Y |
|---|---|---|---|
| 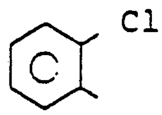 | N | Cl | Cl |
| 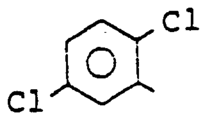 | N | Cl | Cl |
| 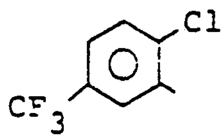 | N | Cl | Cl |
| 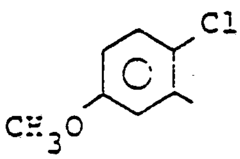 | N | Cl | Cl |
| 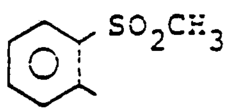 | N | Cl | Cl |
| 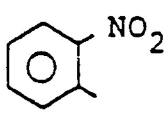 | N | Cl | Cl |
| 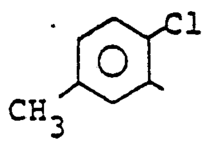 | N | Cl | Cl |
| 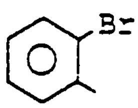 | N | Cl | Cl |
| 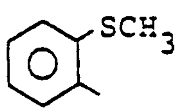 | N | Cl | Cl |

Table I (continued)

| A | Z | X | Y |
|---|---|---|---|
| 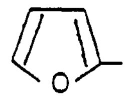 | N | Cl | Cl |
| 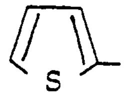 | N | Cl | Cl |
| 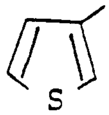 | N | Cl | Cl |
| 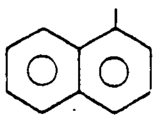 | N | Cl | Cl |
| 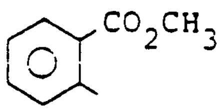 | N | Cl | Cl |
| 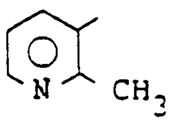 | N | Cl | Cl |
| 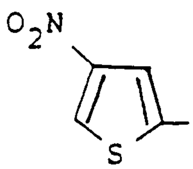 | N | Cl | Cl |
| 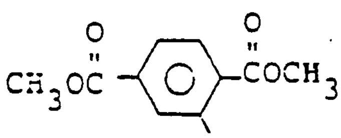 | N | Cl | Cl |
| 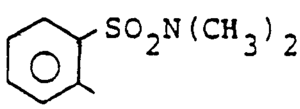 | N | Cl | Cl |
| 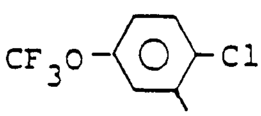 | N | Cl | Cl |
| 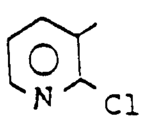 | N | Cl | Cl |

Table I (continued)

| A | Z | X | Y |
|---|---|---|---|
| 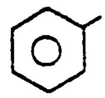 | CH | Cl | Cl |
| 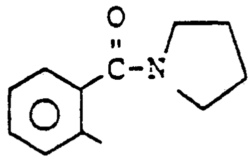 | CH | Cl | Cl |
| 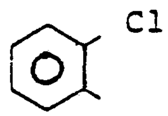 | CH | Cl | Cl |
| 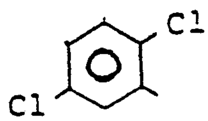 | CH | Cl | Cl |
| 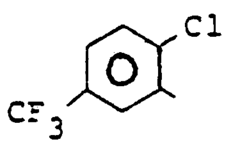 | CH | Cl | Cl |
| 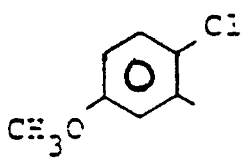 | CH | Cl | Cl |
| 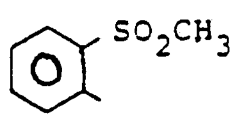 | CH | Cl | Cl |
| 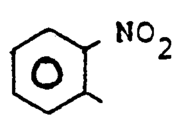 | CH | Cl | Cl |
| 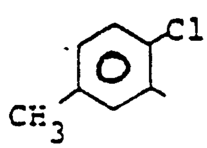 | CH | Cl | Cl |
| 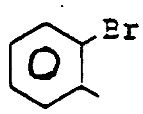 | CH | Cl | Cl |
| 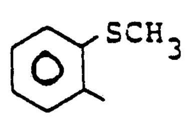 | CH | Cl | Cl |

Table I (continued)

| A | Z | X | Y |
|---|---|---|---|
| 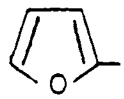 | CH | Cl | Cl |
| 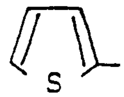 | CH | Cl | Cl |
| 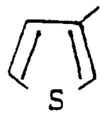 | CH | Cl | Cl |
| 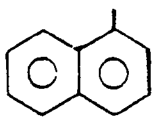 | CH | Cl | Cl |
| 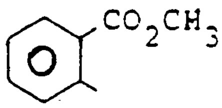 | CH | Cl | Cl |
| 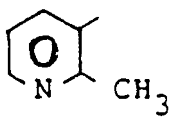 | CH | Cl | Cl |
| 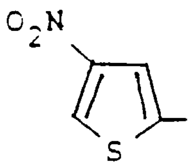 | CH | Cl | Cl |
| 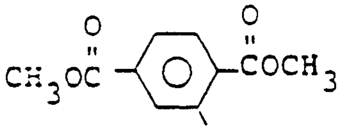 | CH | Cl | Cl |
| 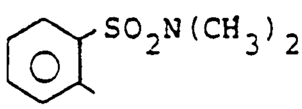 | CH | Cl | Cl |
| 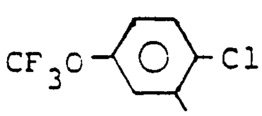 | CH | Cl | Cl |

Table I (continued)

| A | Z | X | Y |
|---|---|---|---|
| 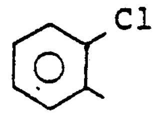 | N | H | Cl |
| 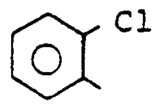 | N | Br | Br |
| 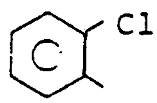 | N | Cl | Br |
| 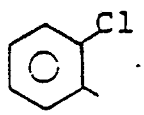 | N | $CH_3$ | Cl |
| 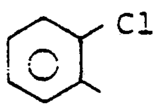 | N | $C_2H_5$ | Cl |
| 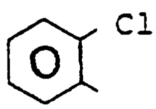 | N | $-CH_2OCH_3$ | Cl |
| 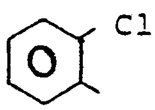 | N | $-CH_2CH_2OCH_3$ | Cl |
| 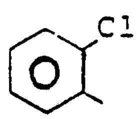 | N | $CF_3$ | Cl |
| 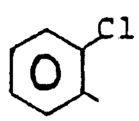 | $C-CH_3$ | Cl | Cl |
| 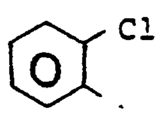 | C-Cl | Cl | Cl |
| 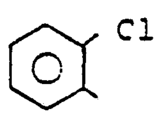 | CH | H | Cl |
| 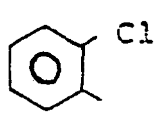 | CH | Br | Br |
| 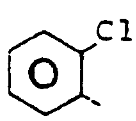 | CH | Cl | Br |

Table I (continued)

| A | Z | X | Y |
|---|---|---|---|
| 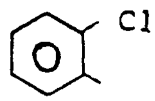 | CH | $CH_3$ | Cl |
| 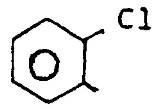 | CH | $C_2H_5$ | Cl |
| 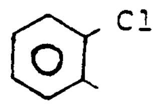 | CH | $CH_2OCH_3$ | Cl |
| 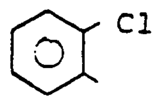 | CH | $CH_2CH_2OCH_3$ | Cl |
| 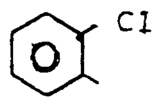 | CH | $CF_3$ | Cl |
| 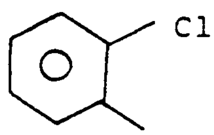 | N | $OCH_3$ | Cl |
| 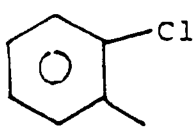 | CH | $OCH_3$ | Cl |

Example 3

N-[(4,6-dimethoxy-1,3,5,-triazin-2-yl)-aminocarbonyl]benzenesulfonamide

1a

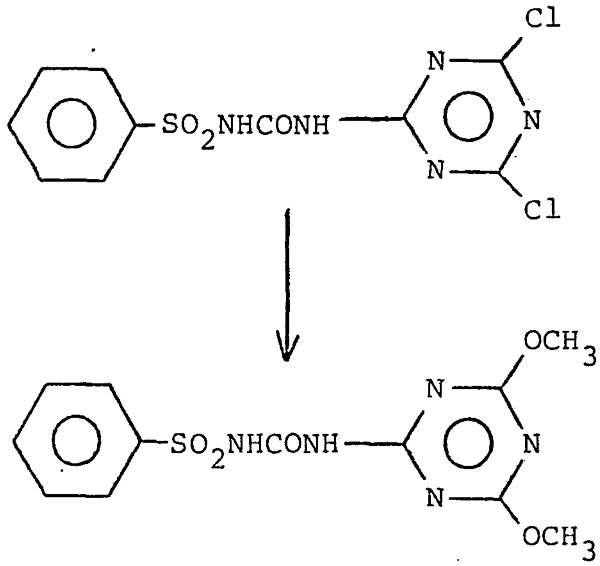

1b

To 0.85g (0.0025 mole) of compound 1a prepared according to Example 1 in 10 ml of methanol, 0.41g (0.0075 mole) of sodium methoxide was added, portionwise. The temperature of the reaction mixture rose to 50°C, and the mixture turned to a clear, slightly yellow solution. Cooling this solution to room temperature yielded a white suspension which was diluted with 30 ml of water. This solution was then acidified to a pH of about 1 with concentrated HCl. A white solid precipitated which was recovered by filtration and dried. The product was 0.6g of compound 1b, a white solid with a melting point of 183-185°.

Example 4

1-/2-([(4,6-dimethoxy-1,3,5-triazin-2-yl)-aminocarbonyl]aminosulfonyl)-benzoyl/pyrrolidine

2a

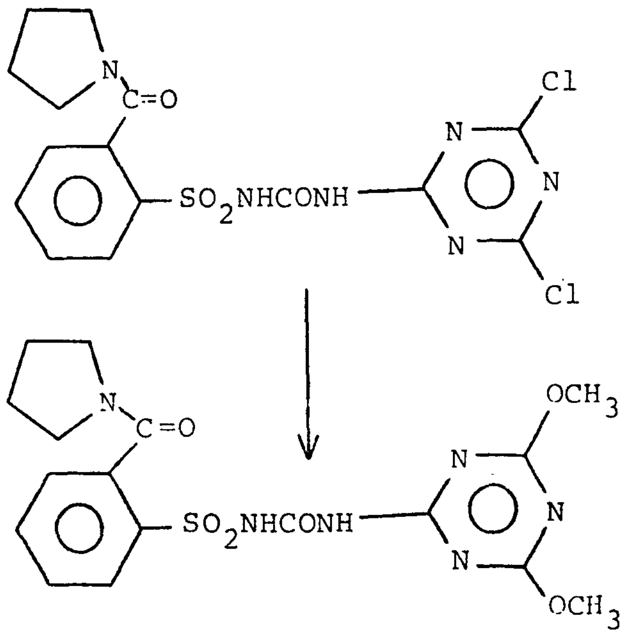

2b

A mixture of 2.2g (0.005 mole) of compound 2a (prepared according to Example 2) in 20 ml of methanol was cooled in an ice bath. Next, a 0.83g portion of sodium methoxide (0.015 mole) was added to the mixture. The temperature of the mixture rose to 20° and a white suspension was produced. This suspension was diluted with 25 ml of water and the small amount of insoluble material was filtered off. The clear filtrate was cooled in an ice-water bath and slowly acidified with concentrated HCl to a pH of about 1. The resulting precipitate was filtered off and dried to provide 1.5g of compound 2b, mp. 158-161°.

Example 5

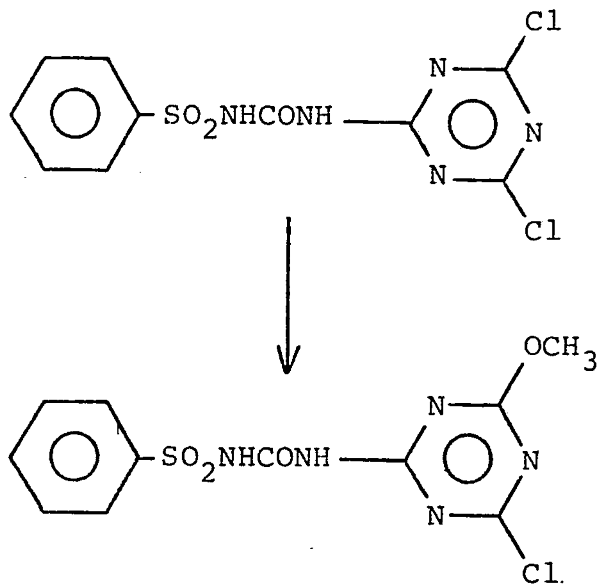

A mixture of 1.75 g (0.005 mole) of N-[(4,6-dichloro-1,3,5-triazin-2-yl)aminocarbonyl]benzenesulfonamide, prepared according to Example 1 in 20 ml of tetrahydrofuran is cooled in an ice bath. Next, a 0.54g portion of sodium methoxide (0.01 mole) is added to the mixture while the temperature of the mixture is maintained at below 5°C. The resulting mixture is warmed to room temperature and diluted with 25 ml water. This solution is then carefully acidified with concentrated HCl to a pH of about 1. The resulting precipitate is filtered off and dried to yield N-[(4-chloro-6-methoxy-1,3,5-triazin-2-yl)-aminocarbonyl] benzenesulfonamide.

According to the process of this invention, the following compounds can be made. This list is exemplary only.

Table II

A-$SO_2$NHCONH 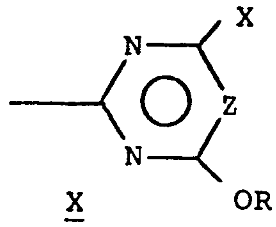

| A | Z | X | R |
|---|---|---|---|
| 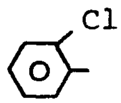 | N | H | $CH_3$ |
| 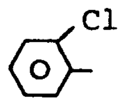 | N | Br | $CH_3$ |
| 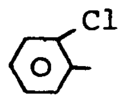 | N | $CH_3$ | $CH_3$ |
| 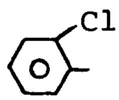 | N | $C_2H_5$ | $CH_3$ |
| 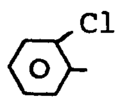 | N | $CH_2OCH_3$ | $CH_3$ |
| 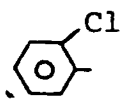 | N | $CH_2CH_2OCH_3$ | $CH_3$ |
| 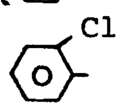 | N | $CF_3$ | $CH_3$ |
| 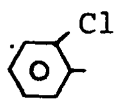 | N | Cl | $-CH_2CO_2CH_3$ |
| 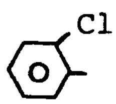 | N | Cl | $-CH(CH_3)CO_2CH_3$ |
| 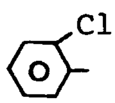 | CH | H | $CH_3$ |
| 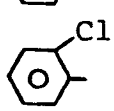 | CH | Br | $CH_3$ |

Table II (continued)

| A | Z | X | R |
| --- | --- | --- | --- |
| 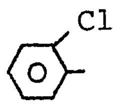 | CH | $CH_3$ | $CH_3$ |
| 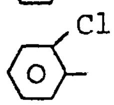 | CH | $C_2H_5$ | $CH_3$ |
| 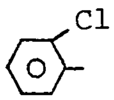 | CH | $CH_2OCH_3$ | $CH_3$ |
| 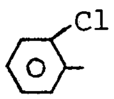 | CH | $CH_2CH_2OCH_3$ | $CH_3$ |
| 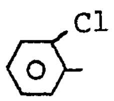 | CH | $CF_3$ | $CH_3$ |
| 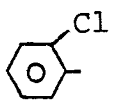 | CH | Cl | $-CH_2CO_2CH_3$ |
| 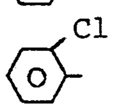 | CH | Cl | $CH_3$<br>$-CHCO_2CH_3$ |
| 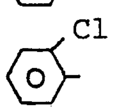 | $CCH_3$ | Cl | $CH_3$ |
| 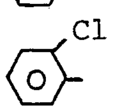 | CCl | Cl | $CH_3$ |
| 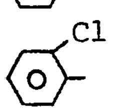 | N | Br | $C_2H_5$ |
| 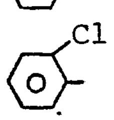 | N | Br | $-CH_2CO_2CH_3$ |
| 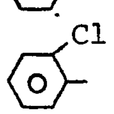 | N | Br | $CH_3$<br>$-CHCO_2CH_3$ |
| 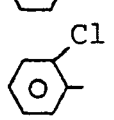 | CH | Br | $C_2H_5$ |

Table II (continued)

| A | Z | X | R |
|---|---|---|---|
| 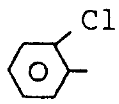 | CH | Br | $-CH_2CO_2CH_3$ |
| 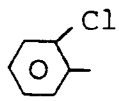 | CH | Br | $-\overset{CH_3}{\overset{\mid}{C}}HCO_2CH_3$ |
| 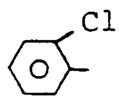 | $CCH_3$ | Br | $CH_3$ |
| 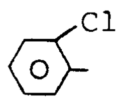 | CCl | Br | $CH_3$ |
| 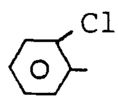 | N | $CH_3O$ | $CH_3$ |
| 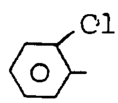 | CH | $CH_3O$ | $CH_3$ |
| 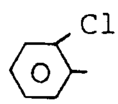 | CCl | $CH_3O$ | $CH_3$ |
| 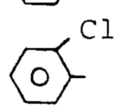 | $CCH_3$ | $CH_3O$ | $CH_3$ |

Table III

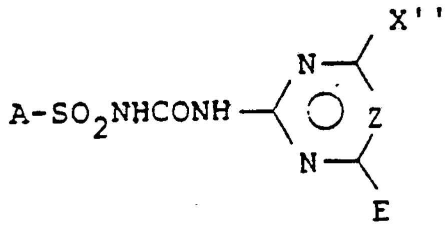

| A | Z | X'' | E |
|---|---|---|---|
| 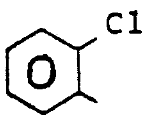 | N | $CH_3$ | $N(CH_3)_2$ |
| 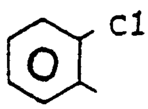 | N | $CH_3$ | $CH_3NC_2H_5$ |
| 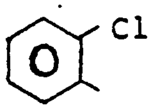 | N | $CH_3$ | $[CH_3(CH_2)_3]_2N$ |
| 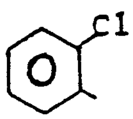 | N | $CH_3$ | $CH_3S$ |
| 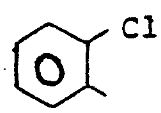 | N | $CH_3$ | $CH_3(CH_2)_5S$ |
| 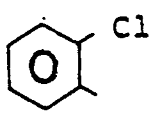 | N | $C_2H_5$ | $N(CH_3)_2$ |
| 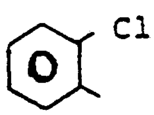 | N | $C_2H_5$ | $SCH_3$ |
| 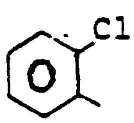 | N | $CF_3$ | $N(CH_3)_2$ |
| 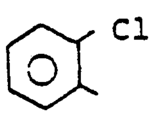 | N | $CF_3$ | $SCH_3$ |
| 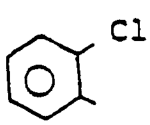 | N | $CH_3OCH_2CH_2$ | $N(CH_3)_2$ |

Table III (continued)

| A | Z | X'' | E |
|---|---|---|---|
| 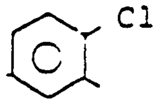 | N | $CH_3OCH_2CH_2$ | $SCH_3$ |
| 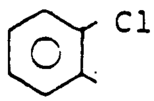 | N | $CH_3OCH_2$ | $N(CH_3)_2$ |
| 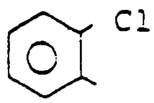 | N | $CH_3OCH_2$ | $SCH_3$ |
| 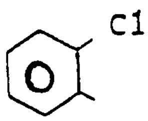 | N | $CH_3O$ | $N(CH_3)_2$ |
| 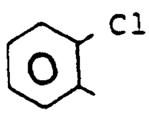 | N | $CH_3O$ | $SCH_3$ |
| 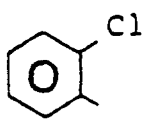 | CH | $CH_3$ | $N(CH_3)_2$ |
| 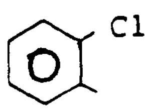 | CH | $CH_3$ | $CH_3NC_2H_5$ |
| 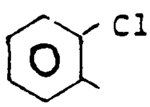 | CH | $CH_3$ | $[CH_3(CH_2)_3]_2N$ |
| 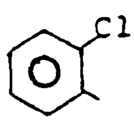 | CH | $CH_3$ | $CH_3S$ |
| 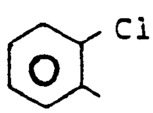 | CH | $CH_3$ | $CH_3(CH_2)_5S$ |
| 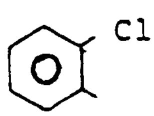 | CH | $C_2H_5$ | $N(CH_3)_2$ |

Table III (continued)

| A | Z | X'' | E |
|---|---|---|---|
| 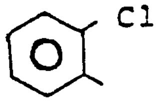 | CH | $C_2H_5$ | $SCH_3$ |
| 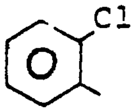 | CH | $CF_3$ | $N(CH_3)_2$ |
| 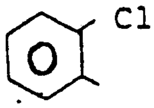 | CH | $CF_3$ | $SCH_3$ |
| 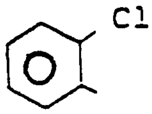 | CH | $CH_3OCH_2CH_2$ | $N(CH_3)_2$ |
| 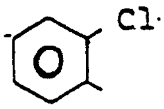 | CH | $CH_3OCH_2CH_2$ | $SCH_3$ |
| 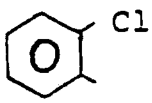 | CH | $CH_3OCH_2$ | $N(CH_3)_2$ |
| 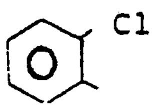 | CH | $CH_3OCH_2$ | $SCH_3$ |
| 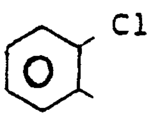 | CH | $CH_3O$ | $N(CH_3)_2$ |
| 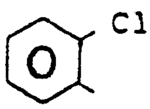 | CH | $CH_3O$ | $SCH_3$ |
| 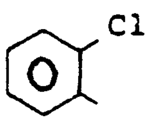 | $CCH_3$ | $CH_3$ | $N(CH_3)_2$ |
| 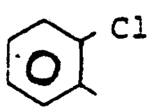 | $CCH_3$ | $CH_3$ | $SCH_3$ |
| 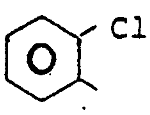 | CCl | $CH_3$ | $N(CH_3)_2$ |
| 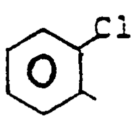 | CCl | $CH_3$ | $SCH_3$ |

Claims:-

1. A process for preparing a herbicidal compound of formula

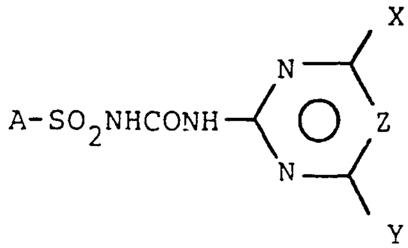

(IV)

(wherein

A is an optionally substituted homocyclic or heterocyclic aromatic group;

X is H; Cl; Br; $C_1$-$C_2$ alkyl; $CF_3$; $CH_3O$; methoxymethyl or $-CH_2CH_2OCH_3$;

Y is Cl or Br; and

Z is CH, N, C-Cl or C-$CH_3$) characterised in that an appropriate aromatic sulfonamide A-$SO_2NH_2$ (wherein A is as defined above) is contacted with an isocyanate of formula

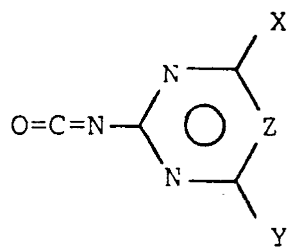

(V)

(wherein X, Y and Z are as defined above).

2. A process as claimed in claim 1 characterised in that the aromatic sulfonamide and the isocyanate are contacted in the presence of a catalytic amount of a base selected from 1,4-diazabicyclo[2.2.2]octane, potassium carbonate, sodium hydride and potassium tert-butoxide.

3. A process as claimed in either of claims 1 and 2 characterized in that in the isocyanate of formula

(V)X is Cl or Br.

4. A process as claimed in any one of the preceding claims characterized in that in the isocyanate of formula(V)X is Cl and Y is Cl.

5. A process as claimed in claim 1 further characterized in that the compound of formula(IV)produced is subsequently subjected to one of the following reaction steps:

(a) to prepare a compound of formula

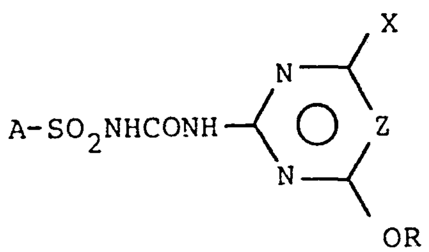

(I)

(wherein A, X and Z are as defined in claim 1 and R is methyl, ethyl, $-CH(CH_3)CO_2CH_3$ or $-CH_2CO_2CH_3$) the compound of formula (IV) (wherein A, X and Z are as defined in claim 1 and Y is as defined in claim 1) is contacted with at least two equivalents of alkoxide ion, -OR (wherein R is as defined above) and the product obtained thereby is acidified;

(b) to prepare a compound of formula

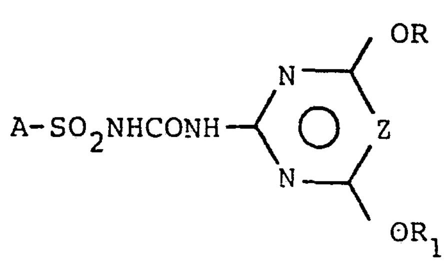

(II)

(wherein A and Z are as defined in claim 1; R is methyl, ethyl, $-CH(CH_3)-CO_2CH_3$ or $-CH_2-CO_2-CH_3$; and $R_1$ is methyl or ethyl) the compound of formula (IV)

(wherein A and Z are as defined in claim 1; Y is as defined in claim 1; and X is Cl or Br) is sequentially contacted with at least two equivalents of alkoxide ion, -OR (wherein R is as defined above), and subsequently with at least one equivalent of alkoxide ion, $-OR_1$ (wherein $R_1$ is as defined above) and the product obtained thereby is acidified;

(c) to prepare a compound of formula

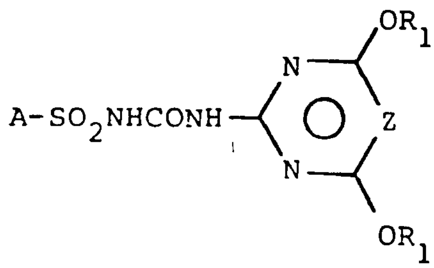

(IIa)

(wherein A and Z are as defined in claim 1 and $R_1$ is methyl or ethyl) the compound of formula (IV) (wherein A and Z are as defined in claim 1; Y is as defined in claim 1; and X is Cl or Br) is contacted with at least three equivalents of alkoxide ion, $-OR_1$ (wherein $R_1$ is as defined above) and the product obtained thereby is acidified; and

(d) to prepare a compound of formula

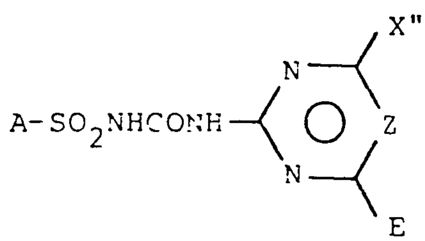

(III)

(wherein A and Z are as defined in claim 1; X" is $C_1$-$C_2$ alkyl, $-CF_3$, 2-methoxyethyl, $-OCH_3$ or methoxymethyl; and E is $-NR_2R_3$ or $C_1$-$C_6$ alkylthio (in which $R_2$ and $R_3$, which may be the same or different, are $C_1$-$C_4$ alkyl)) the compound of formula (IV) (wherein A and Z are as defined in claim 1; Y is as defined in claim

1; and X is X" where X" is as defined above) is contacted with (i) at least one equivalent of $C_1-C_6$ alkyl mercaptan or at least one equivalent of $HNR_2R_3$ (wherein $R_2$ and $R_3$ are as defined above), and (ii) at least two equivalents of base, and the product obtained thereby is acidified.

6. A process as claimed in claim 5 characterized in that in reaction step (a) the compound of formula (IV) is suspended or dissolved in an alkanol solvent of formula ROH (wherein R is as defined in claim 5) in the presence of at least two equivalents of alkoxide ion, -OR (wherein R is defined above).

7. A process as claimed in either of claims 5 or 6 characterized in that in the said compound of formula (I) X is $C_1-C_2$ alkyl and R is methyl or ethyl.

8. A process as claimed in claim 5 characterized in that in reaction step (b) in the alkoxide ion -OR, R is methyl or ethyl.

9. A process as claimed in claim 5 characterized in that in reaction step (d) the $C_1-C_6$ alkyl mercaptam or secondary amine $HNR_2R_3$, is $CH_3SH$, $CH_3CH_2SH$, $(CH_3)_2NH$ or $(CH_3CH_2)_2NH$ and in the compound of formula (IV) contacted therewith X is methyl or ethyl.

10. A process for preparing a compound of formula

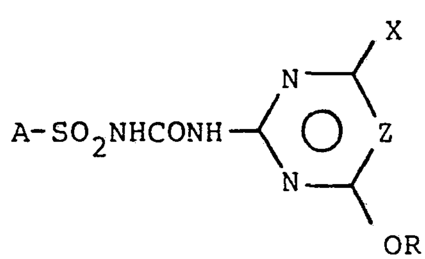

(I)

(wherein

A is an optionally substituted homocyclic or heterocyclic aromatic group;

X is H; Cl; Br; $C_1-C_2$ alkyl; $CH_3O$; $CH_3$; methoxymethyl or methoxyethyl;

Z is CH, N, C-Cl or C-$CH_3$; and

R is methyl, ethyl, $\overset{CH_3}{CH}-CO_2CH_3$ or $CH_2CO_2CH_3$) characterized in that a compound of formula

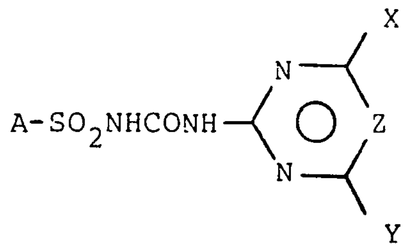

(IV)

(wherein

Y is Cl or Br and A, X and Z are as defined above) is contacted with at least two equivalents of alkoxide ion, -OR (wherein R is as defined above) and the product obtained thereby is acidified.

11. A process for preparing a compound of formula

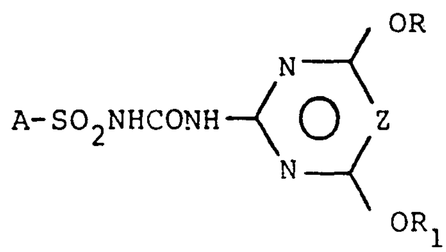

(II)

(wherein

A is an optionally substituted homocyclic or heterocyclic aromatic group;

Z is CH, N, C-Cl or $C-CH_3$;

R is methyl, ethyl, $\overset{CH_3}{CH}-CO_2CH_3$ or $CH_2CO_2CH_3$;

and $R_1$ is methyl or ethyl) characterized in that a compound of formula

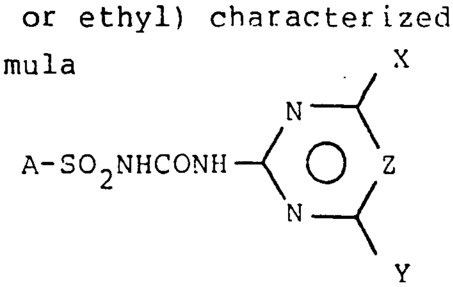

(IV)

(wherein

Y is as defined in claim 1; X is Cl or Br;

and A and Z are as defined above) is sequentially contacted with at least two equivalents of alkoxide ion $-OR_2$ (wherein R is as defined above) and subsequently with at least one equivalent of alkoxide ion, $-OR_1$ (wherein $R_1$ is as defined above) and the product obtained thereby is acidified.

12. A process for preparing a compound of formula

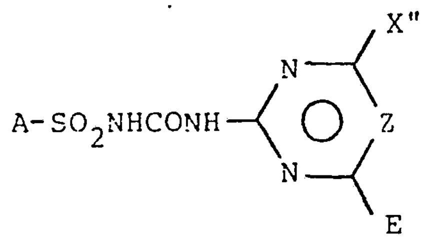

(III)

(wherein

A is an optionally substituted homocyclic or heterocyclic aromatic group;

X" is $C_1$-$C_2$ alkyl, $CF_3$, $CH_3O$, 2-methoxyethyl or methoxymethyl;

E is $-NR_2R_3$ or $C_1$-$C_6$ alkylthio;

$R_2$ and $R_3$ are independently $C_1$-$C_4$alkyl; and

Z is CH, N, C-Cl or C-$CH_3$) characterized in that

(a) a compound of formula

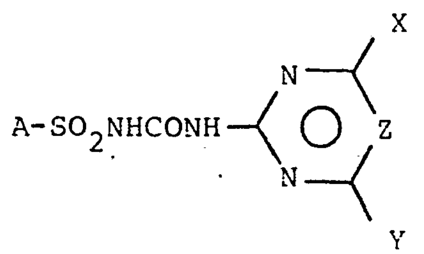

(IV)

(wherein

Y is Cl or Br; X is X" where X" is as defined above; and Z and A are as defined above) is contacted with (i) at least one equivalent

of $C_1$-$C_6$ alkyl mercaptan or at least one equivalent of $HNR_2R_3$ and (ii) at least two equivalents of base; and

(b) the product of step (a) is acidified.

13. A process as claimed in any of the preceding claims characterized in that substituent A is a group selected from optionally substituted phenyl, napthyl, furanyl, thienyl and pyridyl groups.

14. A process as claimed in any of the preceding claims characterized in that substituent A is a group selected from ortho-substituted phenyl, 2-furanyl, 2-thienyl, 3-thienyl, 2-substituted-3-thienyl, 4-substituted-3-thienyl, ortho-substituted pyridyl, ortho-carboxylic acid or carboxylic acid amide substituted phenyl and ortho pyrrolidine carboxamide substituted phenyl groups.

15. A process as claimed in any of the preceding claims characterized in that Z in compounds containing group Z is CH or N.